Europäisches Patentamt

European Patent Office

Office européen des brevets

(11) Publication number: **0 140 386**
**B1**

# (12) EUROPEAN PATENT SPECIFICATION

(45) Date of publication of patent specification: **03.10.90**

(21) Application number: **84113134.5**

(22) Date of filing: **31.10.84**

(51) Int. Cl.⁵: **A 61 K 35/74,** A 61 K 39/10, C 12 P 21/00

(54) Method for the purification of LPF-HA.

(30) Priority: **01.11.83 JP 206598/83**

(43) Date of publication of application:
**08.05.85 Bulletin 85/19**

(45) Publication of the grant of the patent:
**03.10.90 Bulletin 90/40**

(84) Designated Contracting States:
**DE FR GB**

(56) References cited:
**EP-A-0 003 916**

BIOLOGICAL ABSTRACTS, vol. 69, no. 7, 1980, pages 4988,4989, abstract no. 46525; L.I. IRONS et al.: "Isolation of the lymphocytosis promoting factor-hemagglutinin of Bordetella pertussis by affinity chromatography", & BIOCHIM. BIOPHYS. ACTA 580(1), 175, 185, 1979

INFECTION AND IMMUNITY, vol. 31, no. 1, January 1981, pages 495-499; H. ARAI et al.: "Crystallization of pertussigen from Bordetella pertussis"

PROCEEDINGS OF THE NATIONAL ACADEMY OF SCIENCES OF USA, vol. 79, October 1982, pages 6229-6231; R.J. STOCKERT et al.: "IgA interaction with the asialoglycoprotein receptor"

(73) Proprietor: **Juridical Foundation The Chemo-Sero-Therapeutic Research Institute**
**668, Okubo Shimizu-machi**
**Kumamoto-shi Kumamoto-ken (JP)**

(72) Inventor: **Sakuma, Shin**
**2056-32, Nagamine**
**Kumamoto-shi Kumamoto-ken (JP)**
Inventor: **Sakamoto, Kuniaki**
**160-5 Miyukifueda machi**
**Kumamoto-shi Kumamoto-ken (JP)**
Inventor: **Kitagawa, Hisashi**
**4-66, Hieda-machi**
**Kumamoto-shi Kumamoto-ken (JP)**
Inventor: **Sakoh, Mitsuo**
**119-5, Kaminogo-machi**
**Kumamoto-shi Kumamoto-ken (JP)**
Inventor: **Nonaka, Saneo**
**2-21-35, Kurokami**
**Kumamoto-shi Kumamoto-ken (JP)**

(74) Representative: **Vossius & Partner**
**Siebertstrasse 4 P.O. Box 86 07 67**
**D-8000 München 86 (DE)**

Courier Press, Leamington Spa, England.

## Description

The present invention relates to a method for the purification of LPF—HA. More particularly, it relates to a method for producing LPF—HA (Leucocytosis-promoting Factor Hemagglutinin) from culture media of *Bordetella pertussis* in high yield and high purity by an affinity chromatography using a denatured ceruloplasmin as a ligand.

### Technical field

LPF—HA is an active substance produced by *B. pertussis* phase I and phase II strains which is not produced by the non-virulent *B. pertussis* phase III strain or by *Bordetella parapertussis, Bordetella bronchiseptica*. The LPF—HA is also called *B. pertussis* toxin and is a protein having various physiological activities. The main physiological activities are a leucocytosis-promoting activity, an insulin secretion-enhancing activity, a histamine-sensitizing activity, and a hemagglutinating activity. In particular, because of its insulin secretion-enhancing activity, the LPF—HA has been found to be useful for the treatment of diabetes.

Apart from the above physiological activities, LPF—HA has recently been found to play an important role in the prophylaxis of infection with *B. pertussis* and infectious disease thereof and hence is useful as an antigen for prophylaxis of infection by *B. pertussis* [cf. Pittman, M.; Review of Infectious Diseases, *1*, 401—409 (1979), and Sato, Y. et al.; Seminars in Infectious Diseases IV, Bacterial Vaccine, 380—385 (1982)].

Thus, there had been a need to develop an improved method for separating and purifying LPF—HA simply and in large quantities, for the purpose of studying the physiological activities of LPF—HA, of producing a medicine and of producing on an industrial scale a pertussis vaccine having less side effects.

### Prior art

According to known methods, the separation and purification of LPF—HA is carried out by salting out a culture medium of *B. pertussis* with ammonium sulfate, extracting and dialyzing, and then subjecting the thus obtained material to ion exchange chromatography, gel filtration [cf. Arai, H.; Biochimica et Biophysica Acta, *444*, 765 (1976)] or to sucrose concentration gradient centrifugation [cf. Sato, Y.; Infect. Immun., *6*, 897—704 (1972)]. According to such methods, however, the desired LPF—HA which shows a single band in the purification by electrophoresis is very difficult to obtain and its yield is very low.

In order to obtain the desired highly pure LPF—HA in comparatively large amounts it has been proposed to pass the supernatant of a culture medium of *B. pertussis* through a column packed with hydroxyapatite to adsorb LPF—HA thereof, followed by washing, eluting and then subjecting material to affinity chromatography with concanavalin A-Sepharose (Con A-Sepharose, manufactured by Pharmacia) [cf. Yajima, M. et al.; J. Biochem., *83*, 295—303 (1978)]. However, the affinity chromatography using concanavalin A as a ligand not only has an affinity to LPF—HA but can also adsorb saccharides, glycolipids and other glycoproteins, and hence, it adsorbs other pertussis cell components such as F—HA (Filamentous-Hemagglutinin) and cell membrane components, thus causing difficulties in the isolation of the desired highly pure LPF—HA. Consequently, this affinity chromatography is not suitable for the purification of LPF—HA.

Since it has recently been found that human haptoglobin binds specifically to LPF—HA, attempts have been made to purify LPF—HA by an affinity chromatography using as ligand the human haptoglobin instead of the above-mentioned concanavalin [cf. Iron, L. et al.; Biochimica et Biophysica Acta, *580*, 175—185 (1979), and Cowell, J. et al.; Seminars in Infectious Diseases IV, Bacterial Vaccine, 371—379 (1982)]. When using human haptoglobin as ligand, another problem arises, i.e. the necessity of determining the presence of hepatisis virus. This is so, because the human haptoglobin is obtained from human blood, and may thus be contaminated with hepatitis virus and other unknown infectious factors. This problem is also encountered when using animal blood. Unfortunately, however, there is no method for reliably checking the contamination by heptatitis virus. It is also known that the hepatitis virus can be inactivated by heating it at 60°C for 10 to 15 hours. It has been found by the present inventors that when haptoglobin is subjected to such a heat treatment, it looses almost all its afinity to LPF—HA and hence cannot exhibit the desired effect when used in the affinity chromatography.

Moreover, in the case of the purification using hydroxyapatite as mentioned above, treatment on a column packed with hydroxyapatite takes a long time and, as a result, the activity of LPF—HA is reduced. Thus, this method is not suitable either for purifying LPF—HA at low costs and on an industrial scale.

Recently, there has also been proposed a method for isolating LPF—HA which comprises mechanically disrupting *B. pertussis* cells, extracting LPF—HA from the cell debris, subjecting the LPF—HA to ammonium sulfate fractionation, and then, subjecting the thus obtained material to affinity chromatography using as a ligand plasma sialoproteins such as haptoglobin or ceruloplasmin, or sialoproteins such as salivary mucin (cf. British Patent First Publication 2,015,531). However, the ligand specifically disclosed in this British patent publication is merely human haptoglobin, and hence, this method again involves the necessity of ascertaining the absence of hepatitis virus.

Besides, the above British patent publication does not specifically mention ceruloplasmin, and it is unclear therefrom whether or not it is effective. The present inventors have found that when ceruloplasmin is used as such, it is not sufficiently effective in the purification of LPF—HA.

Objects of the Invention

Based on the above technical situation, the present inventors have made intensive investigations for an approved method for isolation and purification of LPF—HA on an industrial scale. They have found that when a denatured ceruloplasmin is used as a ligand in an affinity chromatography, the desired purification of LPF—HA can be achieved, and further that even when it is heat-treated at 60°C for 10 to 15 hours in order to eliminate hepatitis virus, etc., its adsorbability of LPF—HA is not lowered but increased.

The main object of the present invention therefore is to provide an improved method for obtaining *B. pertussis* LPF—HA in high yield and high purity in a single step, wherein the isolation of LPF—HA from culture media of *B. pertussis* is carried out by an affinity chromatography using a denature ceruloplasmin as a ligand. Another object of the invention is to provide a highly pure LPF—HA not contaminated with hepatitis virus or other infectious factors as a consequence of the ligand used in the affinity chromatography. These and other objects and advantages of the present invention will be apparent to persons skilled in the art from the following description.

Detailed Explanation of the Invention

The method for the purification of LPF—HA according to the present invention is characterized in that the starting culture medium obtained by culturing *B. pertussis* in a usual manner is subjected to an affinity chromatography using a denatured ceruloplasmin as a ligand, whereby the endotoxins of *B. pertussis* are removed during the purification of LPF—HA to give a highly purified LPF—HA in high yield.

The starting culture media of *B. pertussis* include culture media obtained by culturing *B. pertussis* phase I (or phase II) strain in a conventional liquid medium, such as Cohen-Wheeler medium or Stainer-Scholte medium, in a usual manner, such as stationary culture or tank culture. Preferably, the culture media are subjected to centrifugation or filtration in order to remove cells. According to the method of the present invention, the culture media can be subjected right away to the affinity chromatography, i.e. without being subjected to various pre-treatments such as salting out, extraction, dialysis, ultracentrifugation, concentration, equilibration, or the like, and hence, the procedure is very simple.

The denatured ceruloplasmin used in the present invention is obtained by denaturing ceruloplasmin of animal origin by various methods, for example, by heating ceruloplasmin at 60 to 85°C for 1 to 24 hours, or by treating ceruloplasmin with a denaturing agent, such as a sulfide (e.g. sodium sulfide, ammonium sulfide, etc.), a reducing sugar (e.g. L-ascorbic acid, D-glucose, D-fructose, maltose, lactose, etc.), a reducing agent (e.g. acetaldehyde, formic acid, oxalic acid, mercaptoethanol, diethyldithiocarbamate, etc.), a cyano compound (e.g. cyanide, sodium thiocyanate, etc.), a chelating agent (e.g. EDTA, nitrotriacetic acid (NTA), triethylenetetraminehexaacetic acid (TTHA), etc.), whereby the copper ions ($Cu^{++}$) contained in ceruloplasmin are reduced or part or all of the copper ions are isolated and removed.

The above denaturing agents may be used alone or in combination of two or more, and denaturation may be carried out after immobilizing ceruloplasmin (i.e., the ligand) on a matrix (i.e. a supporting carrier). Denaturation can easily be carried out by dialyzing one volume of a 0.1 to 0.5 w/v % solution of ceruloplasmin in physiological saline solution against 10 to 200 volumes of the following buffer. That is to say, when using sodium sulfide, ammonium sulfide, or sodium cyanide as the denaturing agent, a 0.01 to 0.1 M phosphate buffer containing 0.01 to 1.0 M of the denaturing agent (pH 4.0—6.0) is used, and dialysis is carried out at 0 to 60°C for 1 to 10 hours. When using L-ascorbic acid or reducing sugars as denaturing agent, a 0.01 to 0.1 M acetate buffer containing 0.01 to 1.0 M of the denaturing agent (pH 4,0—6.0) is used, and dialysis is carried out at 0 to 15°C for 24 to 36 hours. When using acetaldehyde, formic acid, oxalic acid, mercaptoethanol, diethyldithiocarbamate, or the like as the denaturing agent, a 0.01 to 0.1 M phosphate buffer containing 0.01 to 0.1 M of the denaturing agent (pH 6.0—8.0) is used, and the dialysis is carried out 0 to 30°C for 0.5 to 3 hours. When using thiocyanates as denaturing agent, 0.01 to 0.1 M phosphate buffer containing 0.1 to 3.0 M of the denaturing agent (pH 6.0—8.0) is used, and dialysis is carried out at 0 to 30°C for 0.5 to 5.0 hours. When using EDTA, NTA, TTHA as the denaturing agent, a 0.01 to 0.1 M phosphate buffer containing 0.01 to 1.0 M of the denaturing agent (pH 6.0—8.0) is used, and dialysis is carried out at 0 to 30°C for 0.5 to 5.0 hours.

The starting ceruloplasmin is commercially available, or may be obtained by subjecting blood plasma to Cohn's alcohol fractionation as Fraction VI, or may be obtained by separation from human or animal blood and subsequent purification.

The affinity chromatography of culture media of *B. pertussis* with the above denatured ceruloplasmin is usually carried out at a pH range of 4.0 to 10.0 in the following manner.

An affinity gel is prepared by the method of Axe'n et al [cf. Axe'n; Nature, *214*, 1302—1304 (1967)], i.e. by immobilizing the denature ceruloplasmin on a matrix of Sepharose®, agarose, cellulose, or dextran, etc. which are activated with cyanogen bromide. The affinity gel is contacted with the culture medium of *B. pertussis* in a column or in a batch system, whereby LPF—HA contained in the medium is adsorbed onto the gel, followed by washing the gel with an appropriate buffer to remove contaminants and then eluting LPF—HA with an eluent.

According to the column method, the affinity gel is packed into a column, and the starting culture medium of *B. pertussis* is passed through the column at a flow rate of 10 ml/cm²/hour to 500 ml/cm²/hour.

According to the batch method, the culture medium of *B. pertussis* is placed into a vessel, and the affinity gel is directly added thereto. The mixture is stirred for about 30 minutes to about 3 hours, preferably for about one hour.

The amount of the affinity gel is not critical, but usually, 1 ml of affinity gel is used for absorbing 1,000 to 2,000 g of LPF—HA (in protein amount).

Washing of the LPF—HA-absorbed affinity gel is usually carried out with a buffer having a pH 4.0—9.0, and a specific electric conductivity of 10 mS/cm to 150 mS/cm $10^{-1}$ S/m. For example, when using a 0.01 to 0.1 M phosphate buffer (pH 6.0—8.0) containing 0.1 to 1.0 M sodium chloride, washing in the case of the column method is carried out using the buffer in a volume several ten times the volume of the column while in the case of the batch method the buffer is used in a volume several times the volume of the gel. By the washing, the endotoxins of B. pertussis contained in the starting material are effectively removed. This is also one of the characteristics of the present invention, which is superior to the conventional purification methods.

After the above washing step, the LPF—HA adsorbed onto the ligand is eluted in a usual manner using conventional eluents, such as chaotropic salts (e.g. salts which can release chaotropic ions such as $I^-$, $ClO_4^-$, $CF_3COO^-$, $SCN^-$, $CCl_3COO^-$, etc.), ethylene glycol, dioxane, urea, guanidine hydrochloride, EDTA, or the like.

According to the affinity chromatography of the present invention, the desired product can be obtained in high yield, i.e., more than 90% when using starting material having a pH 4.0 to 10.0.

The LPF—HA obtained by the present invention has a high purity of more than 90%, occasionally of more than 95% (in the analysis by electrophoresis) as is shown in Table 1. Besides, the product of the present invention has a specific activity of 135—160 (ELISA unit/mg of protein), which cannot be achieved by the conventional purification methods.

On the contrary, when the purification is carried out using a gel containing haptoglobulin as ligand or a gel containing natural ceruloplasmin as ligand, the product has low purity, e.g. less than 85%, and a specific activity of up to 120—130 (ELISA unit/mg of protein). Thus, it is clear that the specific gel of the present invention shows greatly superior specific adsorbability of LPF—HA in comparison with other known gels. Superiority of the present invention is also clear from the results of biological activity tests of the LPF—HA product, such as the Limurus test and pyrogen test in rabbits, as shown hereinafter. That is, in the case of LPF—HA purified by using a gel containing haptoglobin as ligand, the endotoxin of B. pertussis is not sufficiently removed, and hence, the product shows about 1°C of exotherm, while in the product obtained by the present invention, the undesirable endotoxins are almost completely removed.

Besides, when a haptoglobin is subjected to heat treatment at 60°C for 10 hours in order to remove hepatitis virus and other infectious factors and the heat-treated haptoglobin is used as a ligand for gel, the gel cannot adsorb the LPF—HA completely, and hence, cannot be used for the purification of LPF—HA.

According to the present invention, when using a ceruloplasmin denatured by heat treatment, the specific adsorbability of LPF—HA and capacity of the adsorption are significantly enhanced. Moreover, the hepatitis virus or the like is effectively removed. When using as ligand a ceruloplasmin denatured by 1-ascorbic acid, sodium cyanide etc., the purification capacity of the gel is sufficient, even if the denatured ceruloplasmin is heat-treated 60°C for 10 to 15 hours.

Besides, even when a ceruloplasmin subjected to heat treatment at 60°C for 10 hours is further treated with a denaturing agent (e.g. 1-ascorbic acid or sodium cyanide), the denatured product shows the same properties as the product denatured with the denaturing agent without being subjected to the heat treatment. Thus, since it involves no danger of contamination by hepatitis virus or the like and endotoxins of B. pertussis are sufficiently removed, the present invention is far superior to the method using the known gel comprising haptoglobin as a ligand.

The affinity chromatography of the present invention wherein a denatured ceruloplasmin is used as the ligand can give the desired LPF—HA in high yield and high purity from the starting culture medium of B. pertussis by a simple procedure. Besides, the affinity gel can repeatedly be used several ten times or more, which is advantageous in view of low cost. The method of the present invention is also advantageous in that almost all endotoxins of B. pertussis can be removed. Accordingly, the present invention is very useful as an industrial method for obtaining a highly pure LPF—HA.

The LPF—HA obtained by the present invention is highly pure and does not contain other proteins, lipids or saccharides nor endotoxins, and hence, is useful for the preparation of various reagents utilizing the physiological activities, of a medicine for treating diabetes and of a pertussis vaccine.

The present invention is illustrated by the following Preparations, Examples and Experiments, but should not be construed to be limited thereto.

Preparation 1
Preparation of human ceruloplasmin:

Cohn's Fraction IV-1 obtained by alcohol fraction of human plasma is used as the starting material. The starting material is subjected to the purification method of Morell et al. [cf. J. Biol. Chem., 244, 3494 (1967)] to separate human ceruloplasmin.

That is, the Cohn's Fraction IV-1 is subjected to an ion exchange chromatography with DEAE-Sepharose® CL-6B, and it is subjected to an affinity chromatography with a hemoglobin-Sepharose® gel in order to remove possible contaminants of haptoglobin. Thereafter, the solution is salted out with ammonium sulfate and then dialyzed against 0.025 M acetate buffer (pH 5.25) to obtain crystalline human ceruloplasmin.

Preparation 2

Preparation of denatured ceruloplasmin:

The crystalline ceruloplasmin obtained in Preparation 1 is denatured in a similar manner to the procedure described in Morell et al., Science, *127*, 588 (1963) to prepare various denatured ceruloplasmins.

(1) Ceruloplasmin denatured with sodium cyanide:

A 1 w/v % aqueous solution of crystalline ceruloplasmin (50 ml) is dialyzed against a phosphate buffer (pH 7.4, ionic strength: 0.2) containing 0.05 M sodium cyanide (5.0 liters) at 4°C for 12 hours to give a sodium cyanide-denatured ceruloplasmin which looses 90% of oxidase activity.

(2) Heat treatment of sodium cyanide-denatured ceruloplasmin:

A 1 w/v % aqueous solution of sodium cyanide-denatured ceruloplasmin is prepared in the same manner as described in the above (1). The solution (50 ml) is dialyzed against 0.1 M phosphate buffer (pH 7.4) (5.0 liters). After the dialysis, the denatured ceruloplasmin-containing solution is heated at 60°C for 10 hours.

(3) Ceruloplasmin denatured with L-ascorbic acid:

A 1 w/v % aqueous solution of crystalline ceruloplasmin is dialyzed against an acetate buffer (pH 5.2, ionic strength: 1.2) (5.0 liters) containing L-ascorbic acid (5 mg/ml) at 4°C for 36 hours to give an L-ascorbic acid-denatured ceruloplasmin which show 65.5% oxidase activity.

(4) Ceruloplasmin denatured by heat treatment:

A 0.1 M phosphate buffer (pH 7.5) (100 ml) containing 5 w/v % of a crystalline ceruloplasmin is heat-treated at 60°C for 15 hours.

Preparation 3 Heat-treated ceruloplasmin denatured with L-ascorbic acid:

Cohn's Fraction IV-1 obtained by alcohol fractionation of human plasma is heated on a bath at 60°C for 10 hours. The solution is treated in the same manner as described in Preparation 1 to give a heat-treated crystalline ceruloplasmin.

The heat-treated crystalline ceruloplasmin thus obtained is treated in the same manner as described in Preparation 2-(3) to give a heat-treated ceruloplasmin denatured with L-ascorbic acid.

Preparation 4

Preparation of affinity gel:

The above crystalline ceruloplasmin and various denatured ceruloplasmins (as a ligand) are subjected to a coupling reaction with CNBr-activated Sepharose® 4B (manufactured by Pharmacia) to prepare affinity gels in the following manner.

CNBr-activated Sepharose® 4B (1.5 g) is swollen by being soaked in 1.0 mM hydrochloric acid (3.0 liters) for 15 minutes. Then the 1.0 mM hydrochloric acid is removed by suction on a glass filter (3 G) to give a swollen Sepharose® gel (5.25 ml).

Separately, a ligand (protein amount: 150 mg) is dissolved in 0.1 M sodium carbonate buffer (pH 8.3, 75 ml) containing 0.5 M sodium chloride, and thereto is added the above-prepared swollen Sepharose® gel (5.25 ml). The mixture is gently stirred at room temperature for 2 hours to complete the coupling reaction. After the coupling reaction, the reaction mixture is washed with the same sodium carbonate buffer as above (150 ml) four times, and thereto is added 1.0 M ethanolamine (pH 8.0, 150 ml), and the mixture is again reacted while being gently stirred for 2 hours. After completion of the reaction, the reaction mixture is washed with the same sodium carbonate buffer (150 ml) four times to remove ethanolamine. The resulting gel is washed three times with 0.1 M acetate buffer (pH 8.0) containing 1.0 M sodium chloride (150 ml) and three times with 0.1 M borate buffer (pH 8.0) containing 1.0 M sodium chloride (150 ml). The washings with the acetate buffer and the borate buffer are repeated three times each to give ceruloplasmin-Sepharose® affinity gel and denatured ceruloplasmin-Sepharose® affinity gel.

Example 1

Purification of LPF—HA by column method:

The affinity gel (20 ml) prepared in the above Preparation 3 is packed in a column (28 mm∅ × 150 mm∅), and a supernatant of a culture medium of *B. pertussis* (5.0 liters) containing LPF—HA of 1,400 ELISA unit/ml is passed through the column at room temperature at a flow rate of 150 ml/cm²/hour. Thereafter, a 0.1 M phosphate buffer (pH 7.0, specific electric conductivity: 100 mS/cm, 1,500 ml) containing 1.0 M sodium chloride is passed through the column at the same flow rate as above to wash the column.

After washing, an eluent (100 ml) consisting of 0.1 M phosphate buffer (pH 7.5) containing 1.0 M sodium chloride and 3.0 M sodium thiocyanate is passed through the column at a flow rate of 35.0 ml/cm²/ hour in order to elute LPF—HA.

Table I gives the analytical data of the LPF—HA fraction and experimental data obtained with affinity gels using the denatured ceruloplasmins and undenatured ceruloplasmin and haptoglobin (as the references) as ligands.

As is clear from the results, in case of using various denatured ceruloplasmins of the present invention as the ligand, the LPF—HA obtained has high purity and high specific activity. By contrast, when using undenatured ceruloplasmin as the ligand, the product is of low purity and low specific activity and is obtained in very low yield. Besides, when using known haptoglobin as the ligand, the product is obtained in lower yield and inferior quality. Moroever, when a haptoglobin heat-treated at 60°C for 10 hours is used as the ligand, LPF—HA is not completely adsorbed.

The gel of haptoglobin (ligand) used as the reference is prepared by coupling a commercially available haptoglobin with CNBr-activated Sepharose® 4B in the same manner as described in Preparation 4 above.

Table I

| Kind of ligand for affinity gel | Amount of eluent(ml) | LPF-HA activity[1] x $10^4$ unit/ml | HA value | Specific activity[2] | Purity[3] (%) | Yield (%) |
|---|---|---|---|---|---|---|
| NaCN-denatured ceruloplasmin [Prepn. 2-(1)] | 100 | 6.46 | 512 | 137.7 | 92.3 | 92.3 |
| NaCN-denatured, heat-treated ceruloplamin [Prepn. 2-(2)] | 100 | 6.50 | 512 | 141.6 | 93.0 | 92.9 |
| l-Ascorbic acid-denatured ceruloplasmin [Prepn. 2-(3)] | 100 | 6.58 | 1024 | 138.2 | 93.1 | 94.0 |
| Heat treated ceruloplasmin [Prepn. 2-(4)] | 100 | 6.44 | 512 | 150.0 | 94.6 | 92.0 |
| Heat treated, l-ascorbic acid-denatured ceruloplasmin [Prepn.3] | 100 | 6.94 | 512 | 159.5 | 95.3 | 99.1 |
| Undenatured ceruloplasmin [Prepn. 1] | 100 | 5.46 | 512 | 126.7 | 88.8 | 78.0 |
| Haptoglobin | 100 | 4.50 | 256 | 123.1 | 82.7 | 64.3 |
| Heat treated haptoglobin | 100 | 0 | 0 | 0 | 0 | 0 |

1) Measured by ELISA [cf. Sato et al., Symposium on Toxins, proceeding of the 28th symposium on Toxins, 141-144 (1981)]
2) LPF-HA activity (ELISA unit/ml)/protein content ($\mu$g/ml)
3) Measured by polyacrylamide gel electrophoresis - analysis with densitometer

EP 0 140 386 B1

## Example 2

Purification of LPF—HA by batch method:

Into a 5 liter wide neck flask is charged a supernatant (4.0 liters) of a culture medium of *B. pertussis* containing LPF—HA (1,400 ELISA unit/ml), and thereto is directly added an affinity gel (20 ml). The mixture is gently stirred at room temperature for 1 hour to adsorb LPF—HA onto the affinity gel, and thereafter, the supernatant of a culture medium is removed by suction on a glass filter (G2). The gel remaining on the glass filter is washed with 0.1 M phosphate buffer (pH 7.5, specific electric conductivity: 100 mS/cm) containing 1.0 M sodium chloride (300 ml) and a phosphate buffer (pH 8.0, specific electric conductivity: 100 mS/cm) containing 1.0 M sodium chloride (300 ml). The washings are repeated three times each.

After the washing, LPF—HA is eluted from the gel in the following manner. Onto the gel remaining on the glass filter, an eluent (50 ml) is poured which consists of 0.1 M phosphate buffer (pH 7.5) containing 1.0 M sodium chloride and 3.0 M sodium thiocyanate. After keeping the gel there for 5 minutes, it is sucked to elute LPF—HA. The eluted LPF—HA is dialyzed against 0.01 M phosphate buffer (pH 7.5) to remove sodium thiocyanate. The LPF—HA thus obtained is analyzed in the same manner as in Example 1.

The LPF—HA is subjected to the Limurus test to measure the endotoxin of *B. pertussis*. Moreover, leucocytosis-promoting activity in mice and other physiological activites are measured. These results are shown in Table II and Table III.

As is clear from the results, when using a gel of the denatured ceruloplasmin (as a ligand) of the present invention, a highly pure LPF—HA is obtained in high yield and the endotoxin of *B. pertussis* is almost removed (see Limurus test). By contrast, in the case of the reference gel of haptoglobin (as a ligand), the endotoxin is insufficiently removed. The LPF—HAs thus obtained are subjected to pyrogen test in rabbits. The results show that the LPF—HAs purified by the gels of the denatured ceruloplasmins (as a ligand) of the present invention are all negative, which means that the endotoxin is sufficiently removed.

Table II

| Kind of ligand for affinity gel | Amount of eluent (ml) | LPF-HA activity $\times 10^4$ unit/ml | HA value | Specific activity | Purity (%) | Yield (%) | Limurus test (W) |
|---|---|---|---|---|---|---|---|
| NaCN-denatured ceruloplasmin | 50 | 11.1 | 1024 | 157.4 | 92.5 | 99.1 | 100 |
| 1-Ascorbic acid-denatured celuroplasmin | 50 | 11.7 | 1024 | 169.6 | 96.8 | 104.5 | 100 |
| Heat treated ceruloplasmin | 50 | 11.4 | 1024 | 155.5 | 92.6 | 101.8 | 100 |
| Haptoglobin | 50 | 7.8 | 512 | 125.0 | 80.5 | 70.3 | 100 |

[Note]: The starting supernatant of a culture medium has an endotoxin content of $1.0 \times 10^5$ W by Limurus test.

Table III

| Kind of ligand for affinity gel | Leucocytosis-promoting activity in mice *1) (LPU/ml) | Histamine-sensitizing activity in mice *2) (HSU/ml) | $HSD_{50}$ ( g) | Pyrogen test in rabbits (°C) |
|---|---|---|---|---|
| Starting material (Supernatant of culture medium of B. pertussis) | 15.0 | 3.0 | – | 3.5 |
| LPF-HA purified by heat-treated ceruloplasmin gel | 1238.4 | 385.6 | 0.02 | 0.1 |
| LPF-HA purified by haptoglobin | 675.3 | 225.4 | 0.04 | 1.0 |

*1) and *2): Minimum Requirement of Biological Products, Ministry of Health and Welfare, Japan, #287, 1981.

EP 0 140 386 B1

# EP 0 140 386 B1

**Claims**

1. Method for the production of pure LPF—HA (Leucocytosis-promoting factor hemagglutinin) comprising subjecting a culture medium of *Bordetella pertussis* to an affinity chromatography and eluting LPF—HA adsorbed on the affinity chromatography gel with an eluent, characterized in that the culture medium is subjected to an affinity chromatography using a denatured ceruloplasmin as a ligand for the affinity chromatography gel.

2. The method according to claim 1, wherein the affinity chromatography is carried out on a culture medium having pH 4.0 to 10.0.

3. The method according to claim 1, wherein the denatured ceruloplasmin is obtained by heat treatment of a human or animal-origin ceruloplasmin at 60 to 85°C for 1 to 24 hours.

4. The method according to claim 1, wherein the denatured ceruloplasmin is obtained by treating a human or animal-origin ceruloplasmin with a denaturing agent selected from a sulfide, a reducing sugar, a reducing agent, a cyano compound, and a chelating agent, whereby copper ions are reduced or part or all of the copper ions are removed.

5. The method according to claim 4, wherein the denaturing agent is sodium sulfide, ammonium sulfide, L-ascorbic acid, D-glucose, D-fructose, maltose, lactose, acetaldehyde, formic acid, oxalic acid, mercaptoethanol, diethyldithiocarbamate, sodium cyanide, sodium thiocyanate, EDTA, nitrotriacetic acid, or triethylenetetraminehexaacetic acid.

6. The method according to claim 4, wherein the denaturing agent is sodium cyanide or L-ascorbic acid.

7. The method according to claim 1, wherein the affinity chromatography is carried out by a column method comprising passing a culture medium of *B. pertussis* through a column packed with an affinity chromatography gel using a denatured ceruloplasmin as a ligand at a flow rate of 10 to 500 ml/cm$^2$/hour, washing the column with a buffer having a pH 4.0 to 9.0 and a specific electric conductivity of 10 to 150 mS/cm, (10$^{-1}$S/m) followed by eluting the adsorbed LPF—HA with an eluent selected from chaotropic base, ethylene glycol, dioxane, urea, guanidine hydrochloride, and EDTA.

8. The method according to claim 1, wherein the affinity chromatography is carried out by a batch method comprising stirring a mixture of a culture medium of *B. pertussis* and an affinity chromatography gel using a denatured ceruloplasmin as a ligand for 30 minutes to 3 hours, washing the mixture with a buffer having a pH 4.0 to 9.0 and a specific electric conductivity of 10 to 150 mS/cm (10$^{-1}$S/m), followed by eluting the adsorbed LPF—HA with an eluent selected from chaotropic base, ethylene glycol, dioxane, urea, guanidine hydrochloride, and EDTA.

**Patentansprüche**

1. Verfahren zur Herstellung von reinem LPF—HA ("Leucocytosis-promoting factor hemagglutinin"), wobei man ein Kulturmedium von Bordetella pertussis einer Affinitätschromatographie unterwirft und den LPF—HA, der an das Affinitätschromatographiegel adsorbiert ist, mit einem Elutionsmittel eluiert, dadurch gekennzeichnet, daß man das Kulturmedium einer Affinitätschromatographie mit einem denaturierten Ceruloplasmin als Ligand für das Affinitätschromatographiegel unterwirft.

2. Verfahren nach Anspruch 1, wobei die Affinitätschromatographie mit einem Kulturmedium mit einem pH-Wert von 4,0 bis 10,0 durchgeführt wird.

3. Verfahren nach Anspruch 1, wobei das denaturierte Ceruloplasmin durch eine bei 60 bis 85°C durchgeführte 1 bis 24-stündige Wärmebehandlung eines menschlichen oder von einem Tier stammenden Ceruloplasmin erhalten wird.

4. Verfahren nach Anspruch 1, wobei das denaturierte Ceruloplasmin durch Behandlung eines menschlichen oder von einem Tier stammenden Ceruloplasmins mit einem Denaturierungsmittel erhalten wird, das ausgewählt wird aus einem Sulfid, einem reduzierenden Zucker, einem Reduktionsmittel, einer Cyanverbindung und einem Chelatbildner, wobei Kupferionen reduziert oder teilweise oder vollständig entfernt werden.

5. Verfahren nach Anspruch 4, wobei das Denaturierungsmittel Natriumsulfid, Ammoniumsulfid, L-Ascorbinsäure, D-Glucose, D-Fructose, Maltose, Lactose, Acetaldehyd, Ameisensäure, Oxalsäure, Mercaptoethanol, Diethyldithiocarbamat, Natriumcyanid, Natriumthiocyanat, EDTA, Nitrotriessigsäure oder Triethylentetraminhexaessigsäure ist.

6. Verfahren nach Anspruch 4, wobei das Denaturierungsmittel Natriumcyanid oder L-Ascorbinsäure ist.

7. Verfahren nach Anspruch 1, wobei die Affinitätschromatographie nach einem Säulenverfahren durchgeführt wird, in dem man ein Kulturmedium von B. pertussis mit einer Fließgeschwindigkeit von 10 bis 500 ml/cm$^2$/Std. über eine Säule gibt, die ein unter Verwendung eines denaturierten Ceruloplasmins als Ligand hergestelltes Affinitätschromatographiegel enthält, die Säule mit einem Puffer mit einem pH-Wert von 4,0 bis 9,0 und einer spezifischen elektrischen Leitfähigkeit von 10 bis 150 mS/cm (10$^{-1}$ S/m) wäscht und anschließend das adsorbierte LPF—HA mit einem Elutionsmittel eluiert, das ausgewählt wird aus einer chaotropischen Base, Ethylenglykol, Dioxan, Harnstoff, Guanidinhydrochlorid und EDTA.

8. Verfahren nach Anspruch 1, wobei die Affinitätschromatographie nach einem Batch-Verfahren

11

durchgeführt wird, in dem man ein Gemisch eines Kulturmedium von B. pertussis und eines Affinitätschromatographiegels, das unter Verwendung von denaturiertem Ceruloplasmin als Ligand hergestellt wurde, 30 Minuten bis 3 Stunden rührt, das Gemisch mit einem Puffer mit einem pH-Wert von 4,0 bis 9,0 und einer spezifischen elektrischen Leitfähigkeit von 10 bis 150 mS/cm ($10^{-1}$ S/m) wäscht, und anschließend das adsorbierte LPF—HA mit einem Elutionsmittel eluiert, das ausgewählt ist aus einer chaotropischen Base, Ethylenglykol, Dioxan, Harnstoff, Guanidhydrochlorid und EDTA.

## Revendications

1. Procédé de production de LPF—HA (Leucocytosis-promoting factor hemagglutinin, hémagglutinine facteur promoteur de leucocytose) pure consistant à soumettre un milieu de culture de *Bordetella pertussis* à une chromatographie d'affinité et à éluer la LPF—HA adsorbée sur le gel de chromatographie d'affinité avec un éluant, caractérisé en ce qu'il consiste à soumettre le milieu de culture à une chromatographie d'affinité utilisant une céruléoplasmine dénaturée comme ligand du gel de chromatographie d'affinité.

2. Procédé selon la revendication 1, dans lequel la chromatographie d'affinité est effectuée sur un milieu de culture ayant un pH de 4,0 à 10,0.

3. Procédé selon la revendication 1, dans lequel la céruléoplasmine dénaturée est obtenue par traitement thermique d'une céruléoplasmine d'origine humaine ou animale entre 60 et 85°C pendant 1 à 24 heures.

4. Procédé selon la revendication 1, dans lequel la céruléoplasmine dénaturée est obtenue par traitement d'une céruléoplasmine d'origine humaine ou animale avec un agent dénaturant choisi parmi un sulfure, un sucre réducteur, un agent réducteur, un composé cyano et un agent chélatant, pour que les ions cuivre soient réduits ou qu'une partie ou la totalité des ions cuivre soit éliminée.

5. Procédé selon la revendication 4, dans lequel l'agent dénaturant est le sulfure de sodium, le sulfure d'ammonium, l'acide L-ascorbique, le D-glucose, le D-fructose, le maltose, le lactose, l'acétaldéhyde, l'acide formique, l'acide oxalique, le mercaptoéthanol, le dithiocarbamate de diéthyle, le cyanure de sodium, le thiocyanate de sodium, l'EDTA, l'acide nitrotriacétique ou l'acide triéthylènetétraaminehexaacétique.

6. Procédé selon la revendication 4, dans lequel l'agent dénaturant est le cyanure de sodium ou l'acide L-ascorbique.

7. Procédé selon la revendication 1, dans lequel la chromatographie d'affinité est effectuée selon un procédé en colonne comprenant le passage d'un milieu de culture de *B. pertussis* à travers une colonne garnie d'un gel de chromatographie d'affinité utilisant une céruléoplasmine dénaturée comme ligand à un débit de 10 à 500 ml/cm²/h, lavage de la colonne avec un tampon ayant un pH de 4,0 à 9,0 et une conductivité électrique spécifique de 10 à 150 mS/cm ($10^{-1}$ S/m), puis élution de la LPF—HA adsorbée avec un éluant choisi parmi une base chaotrope, l'éthylèneglycol, le dioxanne, l'urée, le chlorhydrate de guanidine et l'EDTA.

8. Procédé selon la revendication 1, dans lequel la chromatographie d'affinité est effectuée selon un procédé discontinu comprenant l'agitation d'un mélange d'un milieu de culture de *B. pertussis* et d'un gel de chromatographie d'affinité utilisant une céruléoplasmine dénaturée comme ligand pendant 30 minutes à 3 heures, lavage du mélange avec un tampon ayant un pH de 4,0 à 9,0 et une conductivité électrique spécifique de 10 à 150 mS/cm ($10^{-1}$ S/m), puis élution de la LPF—HA adsorbée avec un éluant choisi parmi une base chaotrope, l'éthylèneglycol, le dioxanne, l'urée, le chlorhydrate de guanidine et l'EDTA.